# EUROPEAN PATENT APPLICATION

(11) **EP 2 468 757 A1**
(43) Date of publication of application: **27.06.2012**
(21) Application number: 10306368.1
(22) Date of filing: 07.12.2010
(51) Int. Cl.: C07F 3/00

(54) **Novel diazacrown barium precursors for vapor phase deposition of thin films**

(71) Applicant: L'Air Liquide Société Anonyme pour l'Etude et l'Exploitation des Procédés Georges Claude, 75007 Paris (FR)
(72) Inventor: Lachaud, Christophe, 91240 SAINT-MICHEL SUR ORGE (FR); Lahootun, Vanina, 91270 VIGNEUX SUR SEINE (FR); Zauner, Andreas, 78960 VOISINS LE BRETONNEUX (FR)
(74) Representative: Grout de Beaufort, François-Xavier

(57) **Abstract**

A Barium source precursor, comprising a diazacrown ligand, Ba[-N-C(R¹ R²)C(R³ R⁴)-O-C(R⁵ R⁶)C(R⁷R⁸)-O-C(R⁹ R¹⁰)C(R¹¹ R¹²)-]₂ of formula (I): 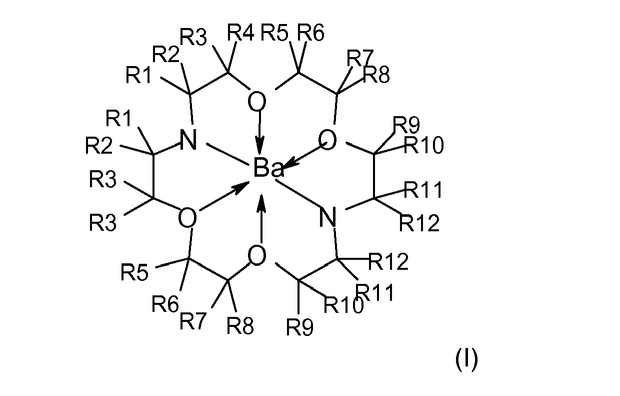 wherein:
R¹-R¹² are organic ligands independently selected in the group consisting of H, a linear or branched function alkyl, aryl, alkenyl, alkylsilyl, alkylamides, alkylsilylamides, alkoxide, fluoroalkyl comprising from 1 to 5 atom of carbon.

## Description

The invention concerns new barium metal-organic compounds required for the vapor phase deposition of barium containing thin films.

DRAM makers are challenged to keep adequate storage capacitance per cell even as the cell size is shrinking. This has to be achieved by moving to higher aspect ratio 3-D structures, and to ultra-high-k (UHK) materials such as perovskites (ABO₃) materials like strontium titanate (SrTiO₃ or STO), barium titanate (BaTiO₃ or BTO) or combination such as (Sr,Ba)TiO₃. Strontium can also be used for the deposition of RuSrOₓ for electrode applications or in flat panel applications (e.g. SrS).

Various barium complexes have been studied in the past for the deposition of thin layer films by (MO)CVD (Metal-Organic Chemical Vapor Deposition), ALD (Atomic Layer Deposition) or other vapor phase deposition methods. These complexes include:
- Amidinate and Guanidinate were disclosed in WO2008/069821. Ba₂(iPr-iPr-iPr-GUA)₄ is an example of this type of precursors. The main disadvantages of this family of precursor are their thermal instability and their lack of volatility. Amidinate and Guanidinate precursors are solid at room temperature with generally a high melting point. Moreover, they tend to form dimeric species.
- β-diketonate ligands generally form solid and non-volatile complexes with Ba/Sr. Sr(thd)₂ is a well-known member of the β-diketonate family. The melting point of this precursor is about 210°C and the boiling point is higher than 350°C under atmospheric pressure. Deposition of SrO₂ using Sr(thd)₂ with O₃ results in the formation of SrO₂ along with Sr₂CO₃. No deposition was observed with Sr(thd)₂ and water. This precursor is not convenient for deposition due to difficulties of vaporization.
- Ba(pivalate)₂ was studied by E. ILJINA et al (Materials science & engineering. B, Solid-state materials for advanced technology 1993, 18, 234-236). This precursor is solid and can be sublimed from 370°C. The vapor pressure at this temperature is only 1,33 Pa. The sublimation leads to nearly 60% of residues.
- Ba(diazacrown ethers) type complexes have been reported as scavenging complexes in the literature.

New barium metal-organic compounds are required for the vapor phase deposition of barium containing thin films. In particular for the deposition of BaSrTiOₓ, BaTiO₃ thin films by (MO)CVD and ALD without the decomposition of the complex. This is due to the high thermal stability of the synthesized alkaline earth metal derivatives.

The present invention concerns a Barium source precursor, comprising a diazacrown ligand, Ba[-N-C(R¹ R²)C(R³ R⁴)-O-C(R⁵ R⁶)C(R⁷ R⁸)-O-C(R⁹ R¹⁰)C(R¹¹ R¹²)-]₂ of formula (I): 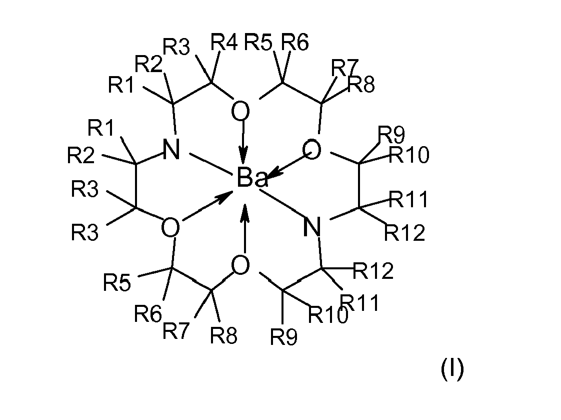 wherein:
R¹-R¹² are organic ligands independently selected in the group consisting of H, C1-C5 linear or branched, alkyl, aryl, alkenyl, alkylsilyl, alkylamides, alkylsilylamides, alkoxide, fluoroalkyl.

In the solid state the precursor may also crystallize with an adduct, A_{X}, where A can be an ethanol or water molecule and x lies between 0 to 2.

This novel family of compounds presents particularly attractive thermal properties. The proposed new precursors present the advantages of:
1) Although being a solid at room temperature the compound sublimes at around 250°C.
2) Having sufficient volatility for vapor phase distribution.
3) Being thermally stable hence allowing proper distribution (gas phase or direct liquid injection) without particles generation.
4) Allowing wide self-limited ALD window.
5) Allowing the deposition of a variety of barium containing films, including ternary or quaternary materials, by using one or a combination of co-reactants (selected among the group comprising O₂, Plasma O₂, H₂O, O₃, TBTDET, TBTDMT, TBTDETCp, TiCl₄, CpTi(OMe)₃, Ta(OEt)₄, Ta(OMe)₅, Ti(OiPr)₄, TDMAT, TDEAT, etc).

This new family of compounds could be used for various other applications (for example in photovoltaic devices) and with various other precursors for ternary or quaternary thin film deposition.

The precursor of the present invention has a sublimation temperature lower than 290 °C.

The described new Ba complexes presents the advantages of being easily synthesized and to be thermally more stable than other currently used Ba precursors for the deposition of high-k materials for the semiconductor industry.

According to another embodiment, the invention concerns a method of forming a barium-containing layer on a substrate surface, the method comprising at least the steps of:
a) providing a vapor comprising at least one precursor compound of the formula (I) as defined above;
b) reacting the vapor comprising the at least one compound of formula (I) with the substrate, according to a method selected from (MO)CVD (Metal-Organic Chemical Vapor Deposition), ALD (Atomic Layer Deposition), PEALD (Plasma Enhanced Atomic Layer Deposition), PECVD (Plasma Enhanced Chemical Vapor Deposition) and derivative vapor phase deposition methods, to form a layer of a at least barium-containing complex on at least one surface of said substrate.

According to another embodiment, the invention concerns a method as defined above further comprising the step:
c) reaction of the complex formed obtained in step b) with at least one reactant selected from another metal source, reducing reactants and/or nitriding reactants and/or oxidizing reactants.

According to another embodiment, the invention concerns a method as defined above, wherein the at least one reactant is selected in the group consisting of, oxygen (O₂), oxygen radicals (for instance O⁻ or OH⁻), for instance generated by a remote plasma, ozone (O₃), moisture (H₂O) and H₂O₂.

According to another embodiment, the invention concerns a method as defined above, wherein the reactants can be introduced simultaneously (chemical vapor deposition), sequentially (atomic layer deposition) or different combinations (one example is to introduce barium source and the other precursor source together in one pulse and oxygen in a separate pulse [modified atomic layer deposition]; another example is to introduce oxygen continuously and to introduce metal source by pulse (pulsed-chemical vapor deposition).

According to another embodiment, the invention concerns a method as defined above, wherein the vapor provided in step a) further comprises one or more metal (M')-organic precursor(s) selected in the group of TBTDET, TBTDMT, (Cp)Ti(OtBu)₃, (Cp)Ti(OiPr)₃, (Cp)Ti(OEt)₃, (Me₅Cp)Ti(OtBu)₃, (Me₅Cp)Ti(OiPr)₃, (Me₅Cp)Ti(OEt)₃, TiCl₄, Ti(MeCp)(OiPr)₃, TDMAT, TDEAT, Ti(OMe)₄, Ta(OEt)₄, Ta(OMe)₅, CpTa(NEt₂)N(tBu), Sr(iPr₃Cp)₂, Ba(iPr₃Cp)₂, to produce thin films containing barium and M'.

According to another embodiment, the invention concerns a method as defined above, wherein the temperature of the substrate is comprised between 310°C and 400°C.

According to another embodiment, the invention concerns a method as defined above, wherein the metal layer is barium titanate.

According to another embodiment, the invention concerns a substrate coated with one or more film monolayers of one or more metals, obtained by a method selected from (MO)CVD (Metal-Organic Chemical Vapor Deposition), ALD (Atomic Layer Deposition), PEALD (Plasma Enhanced Atomic Layer Deposition), PECVD (Plasma Enhanced Chemical Vapor Deposition) and derivative vapor phase deposition methods, wherein at least one precursor is a metal source precursor as defined above.

According to another embodiment, the invention concerns a method of manufacturing a semiconductor device product comprising contacting a semiconductor device substrate with a barium source precursor as defined above, to deposit barium on the substrate.

According to another embodiment, the invention concerns a method of manufacturing a photovoltaic device product comprising contacting a photovoltaic device substrate with a barium source precursor as defined above, to deposit barium on the substrate.

According to another embodiment, the invention concerns a method as defined above, wherein said contacting comprises chemical vapor deposition of barium on the substrate.

According to another embodiment, the invention concerns a method as defined above, wherein said contacting comprises atomic layer deposition of barium on the substrate.

### EXAMPLE:

Here are new barium compounds containing diazacrown ligands according to the present invention:
Ba[-N-C(H₂)C(H₂)-O-C(H₂)C(H₂)-O-C(H₂)C(H₂)-]₂
Ba[-N-C(HMe)C(HMe)-O-C(HMe)C(HMe)-O-C(HMe)C(HMe)-]₂
Ba[-N-C(Me₂)C(Me₂)-O-C(Me₂)C(Me₂)-O-C(Me₂)C(Me₂)-]₂
Ba[-N-C(HMe)C(H₂)-O-C(HMe)C(H₂)-O-C(HMe)C(H₂)-]₂
Ba[-N-C(Me₂)C(HMe)-O-C(Me₂)C(HMe)-O-C(Me₂)C(HMe)-]₂
Ba[-N-C(Me₂)C(H₂)-O-C(Me₂)C(H₂)-O-C(Me₂)C(H₂)-]₂

### Synthesis of the molecules:

### Example 1: Synthesis of Ba[1,10-diaza-18-crown-6] [1].

Ba[1,10-diaza-18-crown-6] was obtained by reaction of barium nitrate with [1,10-diaza-18-crown-6]. [1] was obtained in quantitative yield. The complex formed is a white solid at room temperature that sublimes at 289°C.

### Thermal characterization of the molecules:

All the thermo-gravimetric analyses were performed in an inert atmosphere in order to avoid reaction of the molecules with air and moisture.

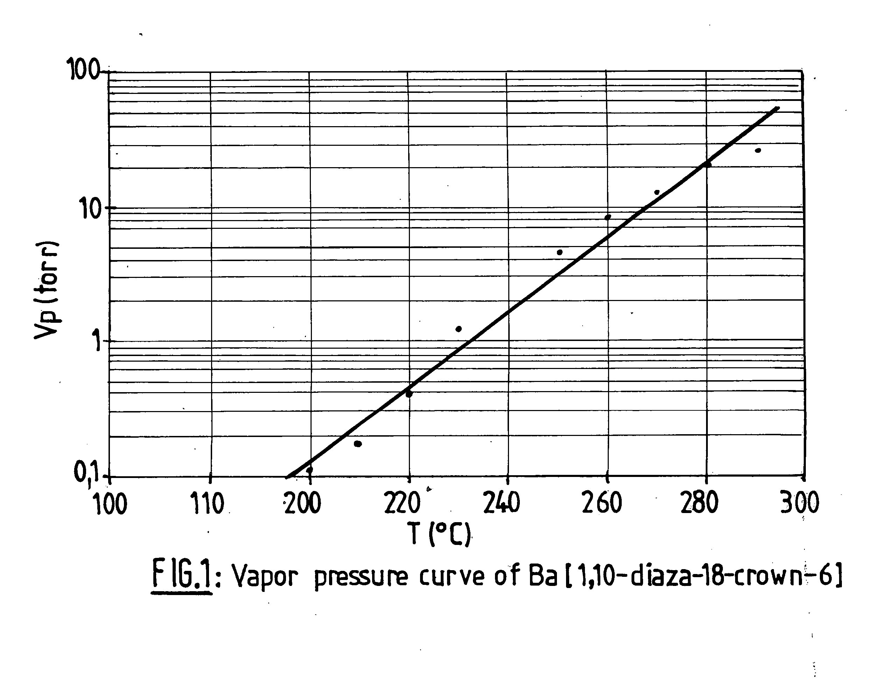

The thermal stability and volatility of molecule [1] was studied by thermogravimetry analysis (TGA). The TGA does not show any indication of decomposition below 300°C. The vapor pressure of this molecule (shown in Figure 1) was evaluated around 133 Pa at 230°C, about ten times higher than Ba(tmhd)₂.

### Method for deposition of barium containing thin films:

The deposition of a barium containing film may be performed in several ways:
1. Vaporization of the barium precursor of the invention as the only metal source.
2. Introduction of several precursors in the vapor phase in a deposition device, wherein said precursors comprise of vaporized barium source, and may comprise another metal source, an oxygen source, a nitrogen source, a carbon source; to form a barium containing film.
3. In one embodiment of the invention, the vaporization of the new precursor can be realized by pressurizing a canister containing the said new precursor. The canister is preferably heated at a temperature which allows a sufficient vapor pressure of the said new precursor. The carrier gas can be selected from Ar, He, H₂, N₂ or mixtures of them. The canister can be heated for instance at temperatures in the range of 80°C to 170°C or higher. The temperature can be adjusted to control the amount of precursor in the gas phase.
4. In another embodiment of the invention, the said new precursor source can be mixed to a solvent or a mixture of solvents, to another metal source or to a mixture of them.
5. In another embodiment of the invention, the said new precursor source is first vaporized. The said new precursor source can be mixed to another metal source. The said mixture can be mixed to a solvent or a mixture of solvents. The said metal source can be mixed to a stabilizer. The said mixture can be introduced in a vaporizer where it is vaporized. The said solvent can be selected from the group consisting of octane, hexane, pentane, tetramethylsilane, mesithylene, etc.
6. In another embodiment of the invention, the said new precursor can be mixed to another precursor and the mixture is vaporized. The said mixture can be added to a solvent or a mixture of solvents. The said solvent can be selected from the group consisting of octane, hexane, pentane, tetramethylsilane, mesithylene, etc.
7. In one embodiment of the invention, the pressure in the said canister is in the range from 0,13 Pa to 13,3*10³ Pa.
8. The said vaporized metal source is introduced into a reaction chamber where it is in contact with a substrate. The substrate can be selected from the group consisting of Si, SiO₂, SiN, SiON, and other metal containing films. The substrate can be heated to an adequate temperature so as to obtain the desired film with a sufficient growth rate and with desired physical state and composition. Typical temperature ranges from 150°C to 450°C. The pressure in the reaction chamber is controlled to obtain the desired metal containing film at sufficient growth rate. Typical pressure ranges from 0,13 Pa to 13,3*10³ Pa.
9. In one embodiment of the invention, the said new precursor source or the mixture described in 3, 4 and 5 is mixed to one or more reactant species prior to the reaction chamber.
10. In one embodiment of the invention, the said new precursor source or the mixture described in 3, 4 and 5 is mixed to one or more reactant species in the reaction chamber.
11. In another embodiment of the invention, the said new precursor and the reactant are introduced sequentially in the reaction chamber. The said new precursor and the reactant species can be introduced simultaneously (chemical vapor deposition), sequentially (atomic layer deposition) or different combinations. One example is to introduce the precursor and another metal source together in one pulse and oxygen in a separate pulse (modified atomic layer deposition); another example is to introduce the reactant (for example oxygen) continuously and to introduce metal source by pulse (pulsed-chemical vapor deposition).
12. In one embodiment of the invention, the reactants are passed through a plasma system localized remotely from the reaction chamber, and decomposed to radicals.
13. In one embodiment of the invention where the targeted metal based film contains oxygen, such as for example BaTiO₃ the said reactant species include an oxygen source which is selected from oxygen (O₂), oxygen radicals (for instance O⁻or OH⁻), for instance generated by a remote plasma, ozone (O₃), moisture (H₂O) and H₂O₂.
14. In one embodiment of the invention one or more reactant species containing nitrogen can be use. The said reactant species are selected from nitrogen (N₂), ammonia, and alkyl derivatives.
15. In one embodiment of the invention, the said metal sources are simultaneously introduced and mixed into the reaction chamber.
16. In another embodiment of the invention, the said metal sources are simultaneously introduced and mixed to reactant species into the reaction chamber.
17. In one embodiment of the invention, the said new precursor is used for atomic layer deposition of barium containing films. The said barium source, the possible said metal source and the reactant species are introduced sequentially in the reaction chamber (atomic layer deposition). The reactor pressure is selected in the range from 0,13 Pa to 13,3*10³ Pa. Preferably, the reactor pressure is comprised between 133 Pa and 1333 Pa. A purge gas is introduced between the metal source pulse and the reactant species pulse. The purge gas can be selected from the group consisting of N₂, Ar, He. The metal source, purge gas and reactant species pulse duration is comprised between 0.1 s and 100 s. Preferably, the pulse duration is comprised between 0.5 s and 10 s.
18. In one embodiment of the invention, the second metal source is selected in the group of TBTDET, TBTDMT, (Cp)Ti(OtBu)₃, (Cp)Ti(OiPr)₃, (Cp)Ti(OEt)₃, (Me₅Cp)Ti(OtBu)₃, (Me₅Cp)Ti(OiPr)₃, (Me₅Cp)Ti(OEt)₃, TiCl₄, Ti(MeCp)(OiPr)₃, TDMAT, TDEAT, Ti(OMe)₄, Ta(OEt)₄, Ta(OMe)₅, CpTa(NEt₂)N(tBu), Sr(iPr₃Cp)₂, Ba(iPr₃Cp)₂ etc.
19. In one embodiment, the reactants can be introduced simultaneously (chemical vapor deposition), sequentially (atomic layer deposition) or different combinations. One example is to introduce barium source and the other precursor source together in one pulse and oxygen in a separate pulse [modified atomic layer deposition]; another example is to introduce oxygen continuously and to introduce metal source by pulse (pulsed-chemical vapor deposition).

## Claims

1. A Barium source precursor, comprising a diazacrown ligand, Ba[-N-C(R¹ R²)C(R³R⁴)-O-C(R⁵ R⁶)C(R⁷ R⁸)-O-C(R⁹ R¹⁰)C(R¹¹R¹²)-]₂ of formula (I): 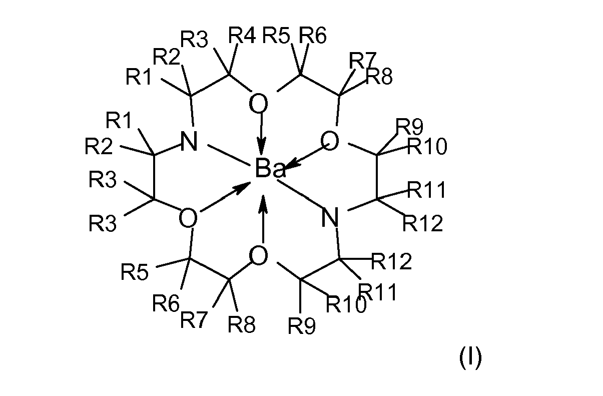 wherein:
R¹-R¹² are organic ligands independently selected in the group consisting of H, a linear or branched function alkyl, aryl, alkenyl, alkylsilyl, alkylamides, alkylsilylamides, alkoxide, fluoroalkyl comprising from 1 to 5 atom of carbon.

2. Method of forming a barium-containing layer on a substrate surface, the method comprising at least the steps of:
a) providing a vapor comprising at least one precursor compound of the formula (I) as defined in claim 1;
b) reacting the vapor comprising the at least one compound of formula (I) with the substrate, according to a method selected from (MO)CVD (Metal-Organic Chemical Vapor Deposition), ALD (Atomic Layer Deposition), PEALD (Plasma Enhanced Atomic Layer Deposition), PECVD (Plasma Enhanced Chemical Vapor Deposition) and derivative vapor phase deposition methods, to form a layer of a at least barium-containing complex on at least one surface of said substrate.

3. Method of claim 2 further comprising the step:
c) reaction of the complex formed obtained in step b) with at least one reactant selected from another metal source, reducing reactants and/or nitriding reactants and/or oxidizing reactants.

4. Method of claim 3 wherein the at least one reactant is selected in the group consisting of, oxygen (O₂), oxygen radicals (for instance O⁻ or OH⁻), for instance generated by a remote plasma, ozone (O₃), moisture (H₂O) and H₂O₂.

5. Method of one of claims 3 or 4 wherein the reactants can be introduced simultaneously (chemical vapor deposition), sequentially (atomic layer deposition) or different combinations such as introducing barium source and the other precursor source together in one pulse and oxygen in a separate pulse (modified atomic layer deposition); or introducing oxygen continuously and to introduce metal source by pulse (pulsed-chemical vapor deposition).

6. Method of one of claims 2 to 5 wherein the vapor provided in step a) further comprises one or more metal (M')-organic precursor(s) selected in the group of TBTDET, TBTDMT, (Cp)Ti(OtBu)₃, (Cp)Ti(OiPr)₃, (Cp)Ti(OEt)₃, (Me₅Cp)Ti(OtBu)₃, (Me₅Cp)Ti(OiPr)₃, (Me₅Cp)Ti(OEt)₃, TiCl₄, Ti(MeCp)(OiPr)₃, TDMAT, TDEAT, Ti(OMe)₄, Ta(OEt)₄, Ta(OMe)₅, CpTa(NEt₂)N(tBu), Sr(iPr₃Cp)₂, Ba(iPr₃Cp)₂, to produce thin films containing barium and M'.

7. Method of one of claims 2 to 6, wherein the temperature of the substrate is comprised between 310°C and 400°C.

8. Method of one of claims 2 to 7, wherein the metal layer is barium titanate.

9. A substrate coated with one or more film monolayers of one or more metals, obtained by a method selected from (MO)CVD (Metal-Organic Chemical Vapor Deposition), ALD (Atomic Layer Deposition), PEALD (Plasma Enhanced Atomic Layer Deposition), PECVD (Plasma Enhanced Chemical Vapor Deposition) and derivative vapor phase deposition methods, wherein at least one precursor is a metal source precursor as defined in claim 1.

10. Method of manufacturing a semiconductor device product comprising contacting a semiconductor device substrate with a barium source precursor as defined in claim 1, to deposit barium on the substrate.

11. Method of manufacturing a photovoltaic device product comprising contacting a photovoltaic device substrate with a barium source precursor as defined in claim 1, to deposit barium on the substrate.

12. Method of one of claims 10 and 11 wherein said contacting comprises chemical vapor deposition of barium on the substrate.

13. Method of one of claims 10 and 11 wherein said contacting comprises atomic layer deposition of barium on the substrate.
